# EUROPEAN PATENT SPECIFICATION

(11) **EP 3 797 145 B1**
(45) Date of publication and mention of the grant of the patent: **30.08.2023**
(21) Application number: 19723115.2
(22) Date of filing: 13.05.2019
(51) Int. Cl.: C10M 105/38, C07D 307/12

(54) **A LUBRICANT COMPRISING 2,5-(BISHYDROXYMETHYL) TETRAHYDROFURAN DIALKANOATES**
SCHMIERMITTEL MIT 2,5-(BISHYDROXYMETHYL)TETRAHYDROFURAN-DIALKANOATEN
LUBRIFIANT COMPRENANT DES DIALCANOATES DE 2,5-(BISHYDROXYMÉTHYL) TÉTRAHYDROFURANE

(30) Priority: 23.05.2018 EP 18173851
(43) Date of publication of application: 31.03.2021
(73) Proprietor: BASF SE, 67056 Ludwigshafen am Rhein (DE)
(72) Inventor: BOHN, Martin Alexander, 67056 Ludwigshafen (DE); GEYER, Karolin, 67056 Ludwigshafen (DE); GRABARSE, Wolfgang, 67056 Ludwigshafen (DE); SCHERER, Markus, 67056 Ludwigshafen (DE); ECORMIER, Muriel, Southampton SO45 3ZG (GB); STRITTMATTER, Jan, 67056 Ludwigshafen (DE)
(74) Representative: BASF IP Association
(86) International application number: PCT/EP2019/062140
(87) International publication number: WO 2019/224027

(56) References cited:
- EP-A1- 3 315 591
- WO-A1-2016/081010
- WO-A1-2016/150786
- Koichiro Naemura ET AL: "Enzyme-catalyzed asymmetric hydrolysis of meso-substrate", CHEMISTRY LETTERS, 1 January 1988 (1988-01-01), pages 1717-1720, XP055603275, Retrieved from the Internet: URL:https://www.journal.csj.jp/doi/pdf/10. 1246/cl.1988.1717 [retrieved on 2019-07-08]

## Description

This invention relates to a lubricant comprising a THF ester of the formula (I) as defined below.

This invention further relates to a use of the THF ester as lubricant; and to a method for reducing friction between moving surfaces comprising the step of contacting the surfaces with the lubricant or with the THF ester.

The commercially available lubricant compositions are produced from a multitude of different natural or synthetic components. To improve the required properties, according to the field of use, further additives are usually added.

The various lubricants must satisfy extremely high criteria such as high viscosity index, good rheological performance, particularly at extreme temperatures, high oxidation stability, good thermal and hydrolytic stability and comparable properties.

Accordingly, high-performance lubricant oil formulations exhibit a special performance profile with respect to shear stability, low-temperature viscosity, long service life, evaporation loss, fuel efficiency, hydrolytic stability, seal compatibility and wear protection.

The commercially available lubricant compositions are produced from a multitude of different natural or synthetic components. To improve the required properties, according to the field of use, further additives are usually added.

The various lubricants must satisfy extremely high criteria such as high viscosity index, good rheological performance, particularly at extreme temperatures, high oxidation stability, good thermal and hydrolytic stability and comparable properties.

Accordingly, high-performance lubricant oil formulations exhibit a special performance profile with respect to shear stability, low-temperature viscosity, long service life, evaporation loss, fuel efficiency, hydrolytic stability, seal compatibility and wear protection.

WO2016/081010 discloses a method for acid-catalyzed acylation of a reduction product of 5-(hydroxylmethyl)-furfural.

WO2016/150786 discloses a use of esters of 2,5-di(hydroxymethyl)tetrahydrofuran as wax for aqueous surfactant compositions.

Naemura et al., Chemistry Letters, 1988, 1717-1720 disclose an enzyme-catalyzed asymmetric hydrolysis of meso-substrates.

EP3315591 discloses a lubricant concentrate comprising an alkoxylated polytetrahydrofuran and a carboxylic acid ester.

The object was solved by a lubricant comprising a THF ester of the formula (I) 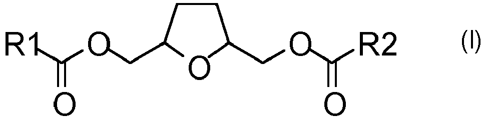 where R¹ and R² are selected independently from C₈-C₁₂ alkyl,
and further comprising
- a base oil selected from mineral oils, polyalphaolefins, polymerized and interpolymerized olefins, alkyl naphthalenes, alkylene oxide polymers, silicone oils, phophate ester and carboxylic acid ester; and/or
- a lubricant additive.

The object was also solved by a use of the THF ester of formula (I) as lubricant.

The object was also solved by a method for reducing friction between moving surfaces comprising the step of contacting the surfaces with the lubricant or with the THF ester of formula (I). The friction may be determined by measuring the friction coefficient at 25% slide roll ratio (SRR) using mini-traction machine (MTM) measurements at 70 °C and 1 GPa.

**R¹ and R²** are selected independently from C₈-C₁₂ alkyl.

R¹ and R² may be linear or branched alkyl. In one form R¹ and R² are linear alkyl. In another form R¹ and R² are branched alkyl. In another form R¹ is branched alkyl and R² is linear alkyl.

In one form R¹ and R² are selected independently from linear C₈-C₁₂ alkyl.

In another form R¹ and R² are selected independently from branched C₈-C₁₂ alkyl.

Suitable R¹ and R² may be independently selected from the group consisting of octyl, nonyl, decyl, undecyl, dodecyl, 2-ethylhexyl, 2-propylheptyl, 2-butyloctyl, and mixtures thereof.

Preferably, R¹ equals R². In another form R¹ and R² are different.

The THF ester of the formula (I) can take the form either of pure cis-isomers or of pure trans-isomers, or of cis/trans-isomer mixtures.

The THF ester of the formula (I) is preferably **liquid,** which usually means that it is liquid at room temperature, e.g. at 25 °C.

The THF ester of the formula (I) may be **clear** liquid at room temperature, e.g. at 25 °C. Typically, in a clear liquid no turbidity is visible.

The THF ester of the formula (I) may have **a pour point** below 25 °C, preferably below 0 °C, and in particular below -15 °C. The pour point may be determined according to ASTM D 97.

The THF ester of the formula (I) may have a **cloud point** of below 25 °C, preferably below 0 °C, and in particular below -15 °C. The cloud point may be determined according to ISO 3015.

The THF ester of the formula (I) may be **miscible** with a polyalphaolefine having a kinematic viscosity at 100 °C of about 6 cSt, where the miscibility is determined in a weight ratio of 50:50 at 25 °C for 24 h.

The THF ester of the formula (I) may have a **viscosity index** of at least 100, preferably at least 120, and in particular of at least 135. The viscosity index may be determined according to ASTM D2270.

The THF ester of the formula (I) may have a **kinematic viscosity at 40°C** from 1 to 100 mm²/s (cSt), preferably from 5 to 50 mm²/s, and in particular from 10 to 20 mm²/s. The kinematic viscosity may be determined according to ASTM D445. In another form the THF ester of the formula (I) may have a kinematic viscosity at 40°C from 200 to 30 000 mm²/s (cSt), preferably from 500 to 15 000 mm²/s, and in particular from 1000 to 5000 mm²/s. The kinematic viscosity may be determined according to ASTM D445.

The THF ester of the formula (I) may have a **kinematic viscosity at 100°C** from 0.1 to 100 mm²/s (cSt), preferably from 0.5 to 30 mm²/s, and in particular from 1 to 10 mm²/s. In another form the THF ester of the formula (I) may have a kinematic viscosity at 100°C from 10 to 8000 mm²/s (cSt), preferably from 30 to 6000 mm²/s, and in particular from 50 to 4000 mm²/s.

The THF ester of the formula (I) is **obtainable** by known methods, for example as described US 2016/0215119, WO 2016/150786, or WO 2016/055196.

2,5-Di(hydroxymethyl)tetrahydrofuran is obtainable for example by hydrogenation of 2,5-di(hydroxymethyl)furan. 2,5-Di(hydroxymethyl)furan can be prepared e.g. starting from fructose by dehydrogenation to 5-hydroxymethylfurfural and subsequent reduction of the formyl group. Consequently, the preparation of 2,5-di(hydroxymethyl)tetrahydrofuran from biogenic sources, starting from corresponding carbohydrates, e.g. starch, cellulose and sugars, is possible.

Alternatively, the preparation of the THF ester of the formula (I) can also take place via the corresponding diesters of 2,5-di(hydroxymethyl)furan, and these are ultimately subjected to a hydrogenation.

Preferably, the starting materials used for the preparation of the THF ester of the formula (I) originate at least partially from a **renewable source,** or their preparation takes place from renewable raw materials. In the context of the invention, renewable sources are understood as meaning natural (biogenic) sources and nonfossil sources, such as natural oil, natural gas or coal. Compounds obtained from renewable sources have a different 14C-to-12C-isotope ratio than compounds obtained from fossil sources, such as natural oil. The THF ester of the formula (I) therefore preferably have a 14C-to-12C-isotope ratio in the range from 0.5×10-12 to 5x10-12.

The **lubricant** further comprises
- a base oil selected from mineral oils, polyalphaolefins, polymerized and interpolymerized olefins, alkyl naphthalenes, alkylene oxide polymers, silicone oils, phosphate ester and carboxylic acid ester; and/or
- a lubricant additive.

In one form the lubribant may comprise at least 10 wt%, preferably at least 30 wt% and in particular at least 60 wt% of the THF ester.

In another form the lubricant may comprise 10 - 99 wt%, preferably 30 - 95 wt% and in particular at least 60 - 95 wt% of the THF ester.

In another form the lubricant may comprise 1 - 90 wt%, preferably 5-50 wt% and in particular 20 - 50 wt% of the base oil.

In another form the lubricant may comprise at least 0.1 wt%, preferably at least 0.5 wt% and in particular at least 1 wt% of the THF ester.

In another form the lubricant may comprise 0.1 - 20 wt%, preferably 0.1 - 150 wt% and in particular at least 0.1 - 10 wt% of the THF ester.

In another form the lubricant may comprise 30 - 99.9 wt%, preferably 50 - 99 wt% and in particular 70 - 95 wt% of the base oil.

The lubricant may comprise up to 20 wt%, preferably up to 15 wt% and in particular up to 10 wt% of the lubricant additive.

In another form the lubricant may comprise 0.1 - 20 wt%, preferably 0.1 - 15 wt% and in particular at least 0.1 - 10 wt% of the lubricant additive.

Lubricants usually refers to composition which are capable of reducing friction between surfaces, such as surfaces of mechanical devices. A mechanical device may be a mechanism consisting of a device that works on mechanical principles. Suitable mechanical device are bearings, gears, joints and guidances. The mechanical device may be operated at temperatures in the range of -30 C to 80 ° C.

The **base oil** may selected from the group consisting of mineral oils (Group I, II or III oils), polyalphaolefins (Group IV oils), polymerized and interpolymerized olefins, alkyl naphthalenes, alkylene oxide polymers, silicone oils, phosphate esters and carboxylic acid esters (Group V oils). Preferably, the base oil is selected from Group I, Group II, Group III base oils according to the definition of the API, or mixtures thereof. Definitions for the base oils are the same as those found in the American Petroleum Institute (API) publication "Engine Oil Licensing and Certification System", Industry Services Department, Fourteenth Edition, December 1996, Addendum 1, December 1998. Said publication categorizes base oils as follows:
a) Group I base oils contain less than 90 percent saturates (ASTM D 2007) and/or greater than 0.03 percent sulfur (ASTM D 2622) and have a viscosity index (ASTM D 2270) greater than or equal to 80 and less than 120.
b) Group II base oils contain greater than or equal to 90 percent saturates and less than or equal to 0.03 percent sulfur and have a viscosity index greater than or equal to 80 and less than 120.
c) Group III base oils contain greater than or equal to 90 percent saturates and less than or equal to 0.03 percent sulfur and have a viscosity index greater than or equal to 120.
d) Group IV base oils contain polyalphaolefins. Polyalphaolefins (PAO) include known PAO materials which typically comprise relatively low molecular weight hydrogenated polymers or oligomers of alphaolefins which include but are not limited to C2 to about C32 alphaolefins with the C8 to about C16 alphaolefins, such as 1-octene, 1-decene, 1-dodecene and the like being preferred. The preferred polyalphaolefins are poly-1-octene, poly-1-decene, and poly-1-dodecene.
e) Group V base oils contain any base oils not described by Groups I to IV. Examples of Group V base oils include alkyl naphthalenes, alkylene oxide polymers, silicone oils, and phosphate esters.

Synthetic base oils include hydrocarbon oils and halo-substituted hydrocarbon oils such as polymerized and interpolymerized olefins (e.g., polypropylenes, propylene-isobutylene copolymers, chlorinated polybutylenes, poly(1-hexenes), poly(1-octenes), poly(1-decenes)); alkylbenzenes (e.g., dodecylbenzenes, tetradecylbenzenes, dinonylbenzenes, di(2-ethylhexyl)benzenes); polyphenyls (e.g., biphenyls, terphenyls, alkylated polyphenols); and alkylated diphenyl ethers and alkylated diphenyl sulfides and derivative, analogs and homologs thereof.

Alkylene oxide polymers and interpolymers and derivatives thereof where the terminal hydroxyl groups have been modified by esterification, etherification, etc., constitute another class of known synthetic base oils. These are exemplified by polyoxyalkylene polymers prepared by polymeriza-tion of ethylene oxide or propylene oxide, and the alkyl and aryl ethers of polyoxyalkylene poly-mers (e.g., methyl-polyiso-propylene glycol ether having a molecular weight of 1000 or diphenyl ether of polyethylene glycol having a molecular weight of 1000 to 1500); and mono- and polycar-boxylic esters thereof, for example, the acetic acid esters, mixed C3-C8 fatty acid esters and C13 oxo acid diester of tetraethylene glycol.

Silicon-based oils such as the polyalkyl-, polyaryl-, polyalkoxy- or polyaryloxysilicone oils and sili-cate oils comprise another useful class of synthetic base oils; such base oils include tetraethyl silicate, tetraisopropyl silicate, tetra-(2- ethylhexyl)silicate, tetra-(4-methyl-2-ethylhexyl) silicate, tetra-(p-tert-butyl-phenyl) silicate, hexa-(4-methyl-2-ethylhexyl)disiloxane, poly(methyl) siloxanes and poly(methylphenyl)siloxanes. Other synthetic base oils include liquid esters of phosphorous-containing acids (e.g., tricresyl phosphate, trioctyl phosphate, diethyl ester of decylphosphonic acid) and polymeric tetrahydrofurans.

Suitable **lubricant additives** may be selected from viscosity index improvers, polymeric thickeners, antioxidants, corrosion inhibitors, detergents, dispersants, anti-foam agents, dyes, wear protection additives, extreme pressure additives (EP additives), anti-wear additives (AW additives), friction modifiers, metal deactivators, pour point depressants.

The **viscosity index improvers** include high molecular weight polymers that increase the relative viscosity of an oil at high temperatures more than they do at low temperatures. Viscosity index improvers include polyacrylates, polymethacrylates, alkylmethacrylates, vinylpyrrolidone/methacrylate copolymers, poly vinylpyrrolidones, polybutenes, olefin copolymers such as an ethylene-propylene copolymer or a styrene-butadiene copolymer or polyalkene such as PIB, styrene/acrylate copolymers and polyethers, and combinations thereof. The most common VI improvers are methacrylate polymers and copolymers, acrylate polymers, olefin polymers and copolymers, and styrenebutadiene copolymers. Other examples of the viscosity index improver include polymethacrylate, polyisobutylene, alpha-olefin polymers, alpha-olefin copolymers (e.g., an ethylenepropylene copolymer), polyalkylstyrene, phenol condensates, naphthalene condensates, a styrenebutadiene copolymer and the like. Of these, polymethacrylate having a number average molecular weight of 10000 to 300000, and alpha-olefin polymers or alpha-olefin copolymers having a number average molecular weight of 1000 to 30000, particularly ethylene- alpha-olefin copolymers having a number average molecular weight of 1000 to 10000 are preferred. The viscosity index increasing agents can be added and used individually or in the form of mixtures, conveniently in an amount within the range of from ≥ 0.05 to ≤ 20.0 % by weight, in relation to the weight of the base stock.

Suitable (polymeric) **thickeners** include, but are not limited to, polyisobutenes (PIB), oligomeric co-polymers (OCPs), polymethacrylates (PMAs), copolymers of styrene and butadiene, or high viscosity esters (complex esters).

**Antioxidants** include phenolic antioxidants such as hindered phenolic antioxidants or non-phenolic oxidation inhibitors.

Useful phenolic antioxidants include hindered phenols. These phenolic antioxidants may be ashless (metal-free) phenolic compounds or neutral or basic metal salts of certain phenolic compounds. Typical phenolic antioxidant compounds are the hindered phenolics which are the ones which contain a sterically hindered hydroxyl group, and these include those derivatives of dihydroxy aryl compounds in which the hydroxyl groups are in the *o*- or *p*-position to each other. Typical phenolic antioxidants include the hindered phenols substituted with alkyl groups having 6 carbon atoms or more and the alkylene coupled derivatives of these hindered phenols. Examples of phenolic materials of this type 2-t-butyl-4-heptyl phenol; 2-t-butyl-4-octyl phenol; 2-t-butyl-4-dodecyl phenol; 2,6-di-t-butyl-4-heptyl phenol; 2,6-di-t-butyl-4-dodecyl phenol; 2-methyl-6-t-butyl-4-heptyl phenol; and 2-methyl-6-t-butyl-4-dodecyl phenol. Other useful hindered mono-phenolic antioxidants may include for example hindered 2,6-di-alkyl-phenolic propionic ester derivatives. Bis-phenolic antioxidants may also be used in combination with the present invention. Examples of ortho-coupled phenols include: 2,2'-bis(4-heptyl-6-t-butyl-phenol); 2,2'-bis(4- octyl-6-t-butyl-phenol); and 2,2'-bis(4-dodecyl-6-t-butylphenol). Para-coupled bisphenols include for example 4,4'-bis(2,6-di-t-butyl phenol) and 4,4'-methylene-bis(2,6-di-t-butyl phenol).

Non-phenolic oxidation inhibitors which may be used include aromatic amine antioxidants and these may be used either as such or in combination with phenolics. Typical examples of non-phenolic antioxidants include: alkylated and non-alkylated aromatic amines such as aromatic monoamines of the formula R⁸R⁹R¹⁰N, where R⁸ is an aliphatic, aromatic or substituted aromatic group, R⁹ is an aromatic or a substituted aromatic group, and R¹⁰ is H, alkyl, aryl or R¹¹S(O)ₓR¹², where R¹¹ is an alkylene, alkenylene, or aralkylene group, R¹² is a higher alkyl group, or an alkenyl, aryl, or alkaryl group, and x is 0, 1 or 2. The aliphatic group R⁸ may contain from 1 to about 20 carbon atoms, and preferably contains from about 6 to 12 carbon atoms. The aliphatic group is a saturated aliphatic group. Preferably, both R⁸ and R⁹ are aromatic or substituted aromatic groups, and the aromatic group may be a fused ring aromatic group such as naphthyl. Aromatic groups R⁸ and R⁹ may be joined together with other groups such as S.

Typical aromatic amines antioxidants have alkyl substituent groups of at least about 6 carbon atoms. Examples of aliphatic groups include hexyl, heptyl, octyl, nonyl, and decyl. Generally, the aliphatic groups will not contain more than about 14 carbon atoms. The general types of amine antioxidants useful in the present compositions include diphenylamines, phenyl naphthylamines, phenothiazines, imidodibenzyls and diphenyl phenylene diamines. Mixtures of two or more aromatic amines are also useful. Polymeric amine antioxidants can also be used. Particular examples of aromatic amine antioxidants useful in the present invention include: p,p'-dioctyldiphenylamine; t-octylphenyl-alpha- naphthylamine; phenyl-alphanaphthylamine; and p-octylphenyl-alpha-naphthylamine. Sulfurized alkyl phenols and alkali or alkaline earth metal salts thereof also are useful antioxidants.

Corrosion inhibitors may include various oxygen-, nitrogen-, sulfur-, and phosphorus-containing materials, and may include metal-containing compounds (salts, organometallics, etc.) and nonmetal-containing or ashless materials. Corrosion inhibitors may include, but are not limited to, additive types such as, for example, hydrocarbyl-, aryl-, alkyl-, arylalkyl-, and alkylaryl-versions of detergents (neutral, overbased), sulfonates, phenates, salicylates, alcoholates, carboxylates, salixarates, phosphites, phosphates, thiophosphates, amines, amine salts, amine phosphoric acid salts, amine sulfonic acid salts, alkoxylated amines, etheramines, polyetheramines, amides, imides, azoles, diazoles, triazoles, benzotriazoles, benzothiadoles, mercaptobenzothiazoles, tolyltriazoles (TTZ-type), heterocyclic amines, heterocyclic sulfides, thiazoles, thiadiazoles, mercaptothiadiazoles, dimercaptothiadiazoles (DMTD-type), imidazoles, benzimidazoles, dithiobenzimidazoles, imidazolines, oxazolines, Mannich reactions products, glycidyl ethers, anhydrides, carbamates, thiocarbamates, dithiocarbamates, polyglycols, etc., or mixtures thereof.

**Detergents** include cleaning agents that adhere to dirt particles, preventing them from attaching to critical surfaces. Detergents may also adhere to the metal surface itself to keep it clean and prevent corrosion from occurring. Detergents include calcium alkylsalicylates, calcium alkylphenates and calcium alkarylsulfonates with alternate metal ions used such as magnesium, barium, or sodium. Examples of the cleaning and dispersing agents which can be used include metal-based detergents such as the neutral and basic alkaline earth metal sulphonates, alkaline earth metal phenates and alkaline earth metal salicylates alkenylsuccinimide and alkenylsuccinimide esters and their borohydrides, phenates, salienius complex detergents and ashless dispersing agents which have been modified with sulphur compounds. These agents can be added and used individually or in the form of mixtures, conveniently in an amount within the range of from ≥ 0.01 to ≤ 1.0 % by weight in relation to the weight of the base stock; these can also be high total base number (TBN), low TBN, or mixtures of high/low TBN.

**Dispersants** are lubricant additives that help to prevent sludge, varnish and other deposits from forming on critical surfaces. The dispersant may be a succinimide dispersant (for example N-substituted long chain alkenyl succinimides), a Mannich dispersant, an ester-containing dispersant, a condensation product of a fatty hydrocarbyl monocarboxylic acylating agent with an amine or ammonia, an alkyl amino phenol dispersant, a hydrocarbyl-amine dispersant, a polyether dispersant or a polyetheramine dispersant. In one embodiment, the succinimide dispersant includes a polyisobutylene-substituted succinimide, wherein the polyisobutylene from which the dispersant is derived may have a number average molecular weight of about 400 to about 5000, or of about 950 to about 1600. In one embodiment, the dispersant includes a borated dispersant. Typically, the borated dispersant includes a succinimide dispersant including a polyisobutylene succinimide, wherein the polyisobutylene from which the dispersant is derived may have a number average molecular weight of about 400 to about 5000. Borated dispersants are described in more detail above within the extreme pressure agent description.

**Anti-foam agents** may be selected from silicones, polyacrylates, and the like. The amount of anti-foam agent in the lubricant compositions described herein may range from ≥ 0.001 wt.-% to≤ 0.1 wt.-% based on the total weight of the formulation. As a further example, an anti-foam agent may be present in an amount from about 0.004 wt.-% to about 0.008 wt.-%.

Suitable **extreme pressure agent** is a sulfur-containing compound. In one embodiment, the sulfur-containing compound may be a sulfurised olefin, a polysulfide, or mixtures thereof.

Examples of the sulfurised olefin include a sulfurised olefin derived from propylene, isobutylene, pentene; an organic sulfide and/or polysulfide including benzyldisulfide; bis-(chlorobenzyl) disulfide; dibutyl tetrasulfide; di-tertiary butyl polysulfide; and sulfurised methyl ester of oleic acid, a sulfurised alkylphenol, a sulfurised dipentene, a sulfurised terpene, a sulfurised Diels-Alder adduct, an alkyl sulphenyl N'N- dialkyl dithiocarbamates; or mixtures thereof. In one embodiment, the sulfurised olefin includes a sulfurised olefin derived from propylene, isobutylene, pentene or mixtures thereof. In one embodiment the extreme pressure additive sulfur-containing compound includes a dimercaptothiadiazole or derivative, or mixtures thereof. Examples of the dimercaptothiadiazole include compounds such as 2,5-dimercapto-1,3,4-thiadiazole or a hydrocarbyl-substituted 2,5-dimercapto-1,3,4-thiadiazole, or oligomers thereof. The oligomers of hydrocarbyl-substituted 2,5-dimercapto-1,3,4-thiadiazole typically form by forming a sulfur-sulfur bond between 2,5-dimercapto-1,3,4-thiadiazole units to form derivatives or oligomers of two or more of said thiadiazole units. Suitable 2,5-dimercapto-1,3,4-thiadiazole derived compounds include for example 2,5-bis(tert-nonyldithio)-1,3,4-thiadiazole or 2-tert-nonyldithio-5-mercapto-1,3,4-thiadiazole. The number of carbon atoms on the hydrocarbyl substituents of the hydrocarbyl-substituted 2,5-dimercapto-1,3,4-thiadiazole typically include 1 to 30, or 2 to 20, or 3 to 16. Extreme pressure additives include compounds containing boron and/or sulfur and/or phosphorus. The extreme pressure agent may be present in the lubricant compositions at 0 wt.-% to about 20 wt.-%, or at about 0.05 wt.-% to about 10.0 wt.-%, or at about 0.1 wt.-% to about 8 wt.-% of the lubricant composition.

Examples of **anti-wear additives** include organo borates, organo phosphites such as didodecyl phosphite, organic sulfur-containing compounds such as sulfurized sperm oil or sulfurized terpenes, zinc dialkyl dithiophosphates, zinc diaryl dithiophosphates, phosphosulfurized hydrocarbons and any combinations thereof.

**Friction modifiers** may include metal-containing compounds or materials as well as ashless compounds or materials, or mixtures thereof. Metal-containing friction modifiers include metal salts or metal-ligand complexes where the metals may include alkali, alkaline earth, or transition group metals. Such metal-containing friction modifiers may also have low-ash characteristics. Transition metals may include Mo, Sb, Sn, Fe, Cu, Zn, and others. Ligands may include hydrocarbyl derivative of alcohols, polyols, glycerols, partial ester glycerols, thiols, carboxylates, carbamates, thiocarbamates, dithiocarbamates, phosphates, thiophosphates, dithiophosphates, amides, imides, amines, thiazoles, thiadiazoles, dithiazoles, diazoles, triazoles, and other polar molecular functional groups containing effective amounts of O, N, S, or P, individually or in combination. In particular, Mo-containing compounds can be particularly effective such as for example Mo-dithiocarbamates, Mo(DTC), Mo-dithiophosphates, Mo(DTP), Mo-amines, Mo (Am), Mo-alcoholates, Mo- alcohol-amides, and the like.

Ashless friction modifiers may also include lubricant materials that contain effective amounts of polar groups, for example, hydroxyl-containing hydrocarbyl base oils, glycerides, partial glycerides, glyceride derivatives, and the like. Polar groups in friction modifiers may include hydrocarbyl groups containing effective amounts of O, N, S, or P, individually or in combination. Other friction modifiers that may be particularly effective include, for example, salts (both ash-containing and ashless derivatives) of fatty acids, fatty alcohols, fatty amides, fatty esters, hydroxyl-containing carboxylates, and comparable synthetic long-chain hydrocarbyl acids, alcohols, amides, esters, hydroxy carboxylates, and the like. In some instances, fatty organic acids, fatty amines, and sulfurized fatty acids may be used as suitable friction modifiers. Examples of friction modifiers include fatty acid esters and amides, organo molybdenum compounds, molybdenum dialkylthiocarbamates and molybdenum dialkyl dithiophosphates.

Suitable **metal deactivators** include benzotriazoles and derivatives thereof, for example 4- or 5-alkylbenzotriazoles (e.g. triazole) and derivatives thereof, 4,5,6,7-tetrahydrobenzotriazole and 5,5'-methylenebisbenzotriazole; Mannich bases of benzotriazole or triazole, e.g. 1-[bis(2-ethylhexyl) aminomethyl) triazole and 1-[bis(2- ethylhexyl) aminomethyl)benzotriazole; and alkoxy-alkylbenzotriazoles such as 1-(nonyloxymethyl)benzotriazole, 1-(1-butoxyethyl) benzotriazole and 1-(1-cyclohexyloxybutyl) triazole, and combinations thereof. Additional non-limiting examples of the one or more metal deactivators include 1,2,4-triazoles and derivatives thereof, for example 3-alkyl(or aryl)-1, 2,4-triazoles, and Mannich bases of 1,2,4-triazoles, such as 1-[bis(2-ethylhexyl) aminomethy1-1, 2,4-triazole; alkoxyalky1-1, 2,4-triazoles such as 1-(1-butoxyethyl)-1, 2,4-triazole; and acylated 3-amino-1, 2,4-triazoles, imidazole derivatives, for example 4,4'-methylenebis(2-undecyl-5-methylimidazole) and bis[(N-methyl)imidazol-2-yl]carbinol octyl ether, and combinations thereof. Further non-limiting examples of the one or more metal deactivators include sulfur-containing heterocyclic compounds, for example 2-mercaptobenzothiazole, 2,5-dimercapto-1, 3,4-thia-diazole and derivatives thereof; and 3,5-bis[di(2- ethylhexyl) aminomethyl]-1, 3,4-thiadiazolin-2-one, and combinations thereof. Even further non-limiting examples of the one or more metal deactivators include amino compounds, for example salicylidenepropylenediamine, salicylami-noguanidine and salts thereof, and combinations thereof. The one or more metal deactivators are not particularly limited in amount in the composition but are typically present in an amount of from about 0.01 to about 0.1, from about 0.05 to about 0.01, or from about 0.07 to about 0.1, wt.-% based on the weight of the composition. Alternatively, the one or more metal deactivators may be present in amounts of less than about 0.1, of less than about 0.7, or less than about 0.5, wt.-% based on the weight of the composition.

**Pour point depressants** (PPD) include polymethacrylates, alkylated naphthalene derivatives, and combinations thereof. Commonly used additives such as alkylaromatic polymers and polymethacrylates are also useful for this purpose. Typically, the treat rates range from ≥ 0.001 wt.-% to ≤ 1.0 wt.-%, in relation to the weight of the base stock.

**Demulsifiers** include trialkyl phosphates, and various polymers and copolymers of ethylene glycol, ethylene oxide, propylene oxide, or mixtures thereof.

Examples for **lubricants** are axel lubrication, medium and heavy duty engine oils, industrial engine oils, marine engine oils, automotive engine oils, crankshaft oils, compressor oils, refrigerator oils, hydrocarbon compressor oils, very low-temperature lubricating oils and fats, high temperature lubricating oils and fats, wire rope lubricants, textile machine oils, refrigerator oils, aviation and aerospace lubricants, aviation turbine oils, transmission oils, gas turbine oils, spindle oils, spin oils, traction fluids, transmission oils, plastic transmission oils, passenger car transmission oils, truck transmission oils, industrial transmission oils, industrial gear oils, insulating oils, instrument oils, brake fluids, transmission liquids, shock absorber oils, heat distribution medium oils, transformer oils, fats, chain oils, minimum quantity lubricants for metalworking operations, oil to the warm and cold working, oil for water-based metalworking liquids, oil for neat oil metalworking fluids, oil for semi-synthetic metalworking fluids, oil for synthetic metalworking fluids, drilling detergents for the soil exploration, hydraulic oils, in biodegradable lubricants or lubricating greases or waxes, chain saw oils, release agents, molding fluids, gun, pistol and rifle lubricants or watch lubricants and food grade approved lubricants.

The THF ester according to the invention may be used for many purposes in lubricants, e.g. for increasing the viscosity index of the lubricant, for thickening of the lubricant, for improving the coefficient of friction of the lubricant, for reducing wear, or as a base stock for the lubricant.

### Examples

A THF ester of the formula (I) where R¹ and R² are n-octyl was prepred from 2,5-(bishydroxymethyl) tetrahydrofuran ("THF glycol"), which was prepared from renewable resources according to known methods. The THF glycol was esterified according to known methods by reaction with n-nonanoic acid. The resulting THF ester was characterized as follows:
The Cloud Point CP was -29 °C as determined according to ASTM D 7689.

The Pour Point PP was -30 °C as determined according to ASTM D 7346.

The Kinematic Viscosity at 40°C was 14.3 mm²/s, and at 100 °C was 3.6 mm²/s as determined according to ASTM D 445. The visosity index VI was 141.

The Noack volatility test according to ASTM 5800 B at 200°C showed an evaporation loss of 1.9%.

The DSC data showed a peak temperature of 202 °C, which indicated that the compound decomposed only at very high temperature.

The thermogravimetry showed a weight loss of below 0.3 % at temperatures of up to 200 °C, and of -1.6 % at 250 °C.

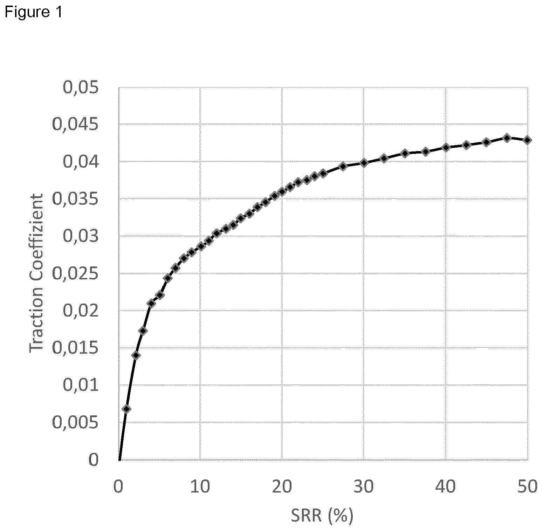

The advantagous friction properties were determined by the friction coefficient at a slide roll ratio (SRR) using mini-traction machine (MTM) measurements (70 °C, 38 N) and are summarized in the traction curve in Figure 1.

## Claims

1. A lubricant comprising a THF ester of the formula (I) 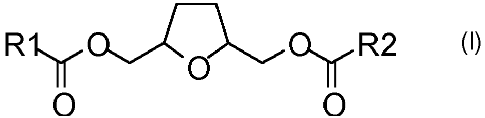 where R¹ and R² are selected independently from C₈-C₁₂ alkyl,
and further comprising
- a base oil selected from mineral oils, polyalphaolefins, polymerized and interpolymerized olefins, alkyl naphthalenes, alkylene oxide polymers, silicone oils, phophate ester and carboxylic acid ester; and/or
- a lubricant additive.

2. The lubricant according to claim 1 where R¹ and R² are linear or branched alkyl.

3. The lubricant according to any of claims 1 or 2 where R¹ and R² are selected independently from C₈ alkyl.

4. The lubricant according to any of claims 1 to 2 where R¹ and R² are independently selected from the group consisting of octyl, nonyl, decyl, undecyl, dodecyl, 2-ethylhexyl, 2-propylheptyl, 2-butyloctyl and mixtures thereof.

5. The lubricant according to any of claims 1 to 4 where R¹ equals R².

6. The lubricant according to any of claims 1 to 5 where the THF ester is miscible with a polyalphaolefine having a kinematic viscosity at 100 °C of about 6 cSt and where the miscibility is determined in a weight ratio of 50:50 at 25 °C for 24 h.

7. The lubricant according to any of claims 1 to 6 where the lubricant comprises at least 1 wt% of the THF ester.

8. The use of a lubricant according to any of claims 1 to 7 for axel lubrication, as medium and heavy duty engine oils, industrial engine oils, marine engine oils, automotive engine oils, crankshaft oils, compressor oils, refrigerator oils, hydrocarbon compressor oils, very low-temperature lubricating oils and fats, high temperature lubricating oils and fats, wire rope lubricants, textile machine oils, refrigerator oils, aviation and aerospace lubricants, aviation turbine oils, transmission oils, gas turbine oils, spindle oils, spin oils, traction fluids, transmission oils, plastic transmission oils, passenger car transmission oils, truck transmission oils, industrial transmission oils, industrial gear oils, insulating oils, instrument oils, brake fluids, transmission liquids, shock absorber oils, heat distribution medium oils, transformer oils, fats, chain oils, minimum quantity lubricants for metalworking operations, oil to the warm and cold working, oil for water-based metalworking liquids, oil for neat oil metalworking fluids, oil for semi-synthetic metalworking fluids, oil for synthetic metalworking fluids, drilling detergents for the soil exploration, hydraulic oils, in biodegradable lubricants or lubricating greases or waxes, chain saw oils, release agents, molding fluids, gun, pistol and rifle lubricants or watch lubricants and food grade approved lubricants.

9. The lubricant according to any of claims 1 to 7 where the lubricant additive is selected from viscosity index improvers, polymeric thickeners, antioxidants, corrosion inhibitors, detergents, dispersants, anti-foam agents, dyes, wear protection additives, extreme pressure additives (EP additives), anti-wear additives (AW additives), friction modifiers, metal deactivators, and pour point depressants.

10. A use of the THF ester of formula (I) as defined in any of the preceding claims as lubricant.

11. The use of the THF ester of formula (I) as defined in any of the preceding claims for increasing the viscosity index of the lubricant, for thickening of the lubricant, for improving the coefficient of friction of the lubricant, for reducing wear, or as a base stock for the lubricant.

12. A method for reducing friction between moving surfaces comprising the step of contacting the surfaces with the lubricant as defined in any of claims 1 to 9 or with the THF ester of formula (I) as defined in any of claims 1 to 6.

## Patentansprüche

1. Schmiermittel, umfassend einen THF-Ester der Formel (I) 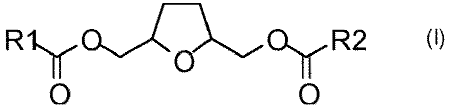 wobei R¹ und R² unabhängig aus C₈-C₁₂-Alkyl ausgewählt sind,
und ferner umfassend
- ein Grundöl, ausgewählt aus Mineralölen, Polyalphaolefinen, polymerisierten und mischpolymerisierten Olefinen, Alkylnaphthalinen, Alkylenoxidpolymeren, Silikonölen, Phosphatestern und Carbonsäureestern; und/oder
- ein Schmiermitteladditiv.

2. Schmiermittel nach Anspruch 1, wobei R¹ und R² für lineares oder verzweigtes Alkyl stehen.

3. Schmiermittel nach Anspruch 1 oder 2, wobei R¹ und R² unabhängig aus C₈-Alkyl ausgewählt sind.

4. Schmiermittel nach Anspruch 1 oder 2, wobei R¹ und R² unabhängig aus der Gruppe bestehend aus Octyl, Nonyl, Decyl, Undecyl, Dodecyl, 2-Ethylhexyl, 2-Propylheptyl, 2-Butyloctyl und Mischungen davon ausgewählt sind.

5. Schmiermittel nach einem der Ansprüche 1 bis 4, wobei R¹ gleich R² ist.

6. Schmiermittel nach einem der Ansprüche 1 bis 5, wobei der THF-Ester mit einem Polyalphaolefin mit einer kinematischen Viskosität bei 100 °C von etwa 6 cSt mischbar ist und wobei die Mischbarkeit in einem Gewichtsverhältnis von 50:50 bei 25 °C über einen Zeitraum von 24 h bestimmt wird.

7. Schmiermittel nach einem der Ansprüche 1 bis 6, wobei das Schmiermittel mindestens 1 Gew.-% des THF-Esters umfasst.

8. Verwendung eines Schmiermittels nach einem der Ansprüche 1 bis 7 für die Achselschmierung, als Motorenöle für mittlere und schwere Belastung, technische Motorenöle, Schiffsmotorenöle, Automobilmotorenöle, Kurbelwellenöle, Kompressoröle, Kältemaschinenöle, Kohlenwasserstoffkompressoröle, Tiefsttemperaturschmieröle und -fette, Hochtemperaturschmieröle und - fette, Drahtseilschmiermittel, Textilmaschinenöle, Kältemaschinenöle, Schmiermittel für die Luft- und Raumfahrt, Flugturbinenöle, Getriebeöle, Gasturbinenöle, Spindelöle, Spinnöle, Traktionsflüssigkeiten, Getriebeöle, Kunststoffgetriebeöle, PKW-Getriebeöle, LKW-Getriebeöle, technische Getriebeöle, technische Zahnradöle, Isolieröle, Instrumentenöle, Bremsflüssigkeiten, Getriebeflüssigkeiten, Stoßdämpferöle, Wärmeverteilungsmediumöle, Transformatoröle, Fette, Kettenöle, Mindestmengenschmiermittel für Metallbearbeitungsvorgänge, Öl für die Warm- und Kaltbearbeitung, Öl für Metallbearbeitungsflüssigkeiten auf Wasserbasis, Öl für Reinölmetallbearbeitungsflüssigkeiten, Öl für halbsynthetische Metallbearbeitungsflüssigkeiten, Öl für synthetische Metallbearbeitungsflüssigkeiten, Bohrdetergentien für die Bodenexploration, Hydrauliköle, biologisch abbaubare Schmiermittel oder Schmierfette oder -wachse, Kettensägenöle, Trennmittel, Formgebungsflüssigkeiten, Schmiermittel für Rohrwaffen, Pistolen und Gewehre oder Schmiermittel für Uhren und für Lebensmittelzwecke zugelassene Schmiermittel.

9. Schmiermittel nach einem der Ansprüche 1 bis 7, wobei das Schmiermitteladditiv aus Viskositätsindexverbesserern, polymeren Verdickern, Antioxidantien, Korrosionsschutzmitteln, Detergentien, Dispergiermitteln, Antischaummitteln, Farbstoffen, Verschleißschutzadditiven, Höchstdruckadditiven (EP-Additiven), Antiverschleißadditiven (AW-Additiven, Reibungsmodifikatoren, Metalldesaktivatoren und Pourpoint-Erniedrigern ausgewählt ist.

10. Verwendung des THF-Esters der Formel (I) gemäß einem der vorhergehenden Ansprüche als Schmiermittel.

11. Verwendung des THF-Esters der Formel (I) gemäß einem der vorhergehenden Ansprüche zur Erhöhung des Viskositätsindex des Schmiermittels, zum Verdicken des Schmiermittels, zur Verbesserung des Reibungskoeffizienten des Schmiermittels, zur Verringerung von Verschleiß oder als Grundstoff für das Schmiermittel.

12. Verfahren zur Verringerung von Reibung zwischen sich bewegenden Oberflächen, bei dem man die Oberflächen mit dem Schmiermittel gemäß einem der Ansprüche 1 bis 9 oder mit dem THF-Ester der Formel (I) gemäß einem der Ansprüche 1 bis 6 in Kontakt bringt.

## Revendications

1. Lubrifiant comprenant un ester de THF de la formule (I) 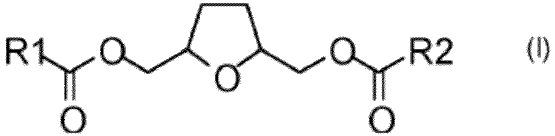 dans laquelle R¹ et R² sont choisis indépendamment parmi alkyle en C₈-C₁₂,
et comprenant en outre
- une huile de base choisie parmi des huiles minérales, des polyalphaoléfines, des oléfines polymérisées et interpolymérisées, des alkylnaphtalènes, des polymères d'oxyde d'alkylène, des huiles de silicone, un ester de phosphate et un ester d'acide carboxylique ; et/ou
- un additif de lubrifiant.

2. Lubrifiant selon la revendication 1 dans lequel R¹ et R² sont alkyle linéaire ou ramifié.

3. Lubrifiant selon l'une quelconque des revendications 1 ou 2 dans lequel R¹ et R² sont choisis indépendamment parmi alkyle en C₈.

4. Lubrifiant selon l'une quelconque des revendications 1 à 2 dans lequel R¹ et R² sont choisis indépendamment dans le groupe constitué par octyle, nonyle, décyle, undécyle, dodécyle, 2-éthylhexyle, 2-propylheptyle, 2-butyloctyle et des mélanges correspondants.

5. Lubrifiant selon l'une quelconque des revendications 1 à 4 dans lequel R¹ est égal à R².

6. Lubrifiant selon l'une quelconque des revendications 1 à 5 dans lequel l'ester de THF est miscible avec une polyalphaoléfine ayant une viscosité cinématique à 100 °C d'environ 6 cSt et dans lequel la miscibilité est déterminée en un rapport en poids de 50 : 50 à 25 °C pendant 24 h.

7. Lubrifiant selon l'une quelconque des revendications 1 à 6 dans lequel le lubrifiant comprend au moins 1 % en poids de l'ester de THF.

8. Utilisation d'un lubrifiant selon l'une quelconque des revendications 1 à 7 pour la lubrification des essieux, comme huiles pour moteurs moyens et lourds, huiles pour moteurs industriels, huiles pour moteurs marins, huiles pour moteurs automobiles, huiles pour vilebrequins, huiles pour compresseurs, huiles pour réfrigérateurs, huiles pour compresseurs à hydrocarbures, huiles et graisses lubrifiantes à très basse température, huiles et graisses lubrifiantes à haute température, lubrifiants pour câbles métalliques, huiles pour machines textiles, huiles pour réfrigérateurs, lubrifiants pour l'aviation et l'aérospatiale, huiles pour turbines d'aviation, huiles pour transmissions, huiles pour turbines à gaz, huiles pour broches, huiles de rotation, fluides de traction, huiles pour transmissions, huiles pour transmissions en plastique, huiles pour transmissions de voitures particulières, huiles pour transmissions de camions, huiles pour transmissions industrielles, huiles pour engrenages industriels, huiles isolantes, huiles pour instruments, fluides de frein, liquides de transmission, huiles pour amortisseurs, huiles pour fluide caloporteur, huiles pour transformateurs, graisses, huiles pour chaînes, lubrifiants en quantité minimale pour opérations de travail des métaux, huile pour le travail à chaud et à froid, huile pour liquides de travail des métaux à base d'eau, huile pour fluides de travail des métaux à base d'huile pure, huile pour fluides semi-synthétiques de travail des métaux, huile pour fluides synthétiques de travail des métaux, détergents de forage pour l'exploration du sol, huiles hydrauliques, dans des lubrifiants biodégradables ou des graisses ou cires lubrifiantes, huiles pour tronçonneuses, agents de démoulage, fluides de moulage, lubrifiants pour fusils, pistolets et carabines ou lubrifiants pour montres et lubrifiants approuvés de qualité alimentaire.

9. Lubrifiant selon l'une quelconque des revendications 1 à 7 dans lequel l'additif de lubrifiant est choisi parmi des agents d'amélioration de l'indice de viscosité, des épaississants polymériques, des antioxydants, des inhibiteurs de corrosion, des détergents, des dispersants, des agents antimousses, des colorants, des additifs de protection contre l'usure, des additifs de pression extrême (additifs EP), des additifs anti-usure (additifs AW), des agents de modification de la friction, des désactivateurs de métaux, et des agents d'abaissement du point d'écoulement.

10. Utilisation de l'ester de THF de formule (I) tel que défini dans l'une quelconque des revendications précédentes en tant que lubrifiant.

11. Utilisation de l'ester de THF de formule (I) tel que défini dans l'une quelconque des revendications précédentes pour l'augmentation de l'indice de viscosité du lubrifiant, pour l'épaississement du lubrifiant, pour l'amélioration du coefficient de friction du lubrifiant, pour la réduction de l'usure, ou en tant qu'une huile de base pour le lubrifiant.

12. Procédé pour la réduction de la friction entre des surfaces mobiles comprenant l'étape de mise en contact des surfaces avec le lubrifiant tel que défini dans l'une quelconque des revendications 1 à 9 ou avec l'ester de THF de formule (I) tel que défini dans l'une quelconque des revendications 1 à 6.
